Europäisches Patentamt

European Patent Office

Office· européen des brevets

(11) Numéro de publication : **0 035 414**
**B1**

(12) **FASCICULE DE BREVET EUROPÉEN**

(45) Date de publication du fascicule du brevet :
01.06.83

(21) Numéro de dépôt : **81400047.7**

(22) Date de dépôt : **15.01.81**

(51) Int. Cl.³ : **A 61 D 7/00**, A 01 K 39/06,
A 61 M 31/00

(54) Dispositif semi-automatique pour le gavage d'animaux.

(30) Priorité : **08.02.80 FR 8002753**

(43) Date de publication de la demande :
**09.09.81 Bulletin 81/36**

(45) Mention de la délivrance du brevet :
**01.06.83 Bulletin 83/22**

(84) Etats contractants désignés :
**AT BE CH DE GB IT LI LU NL SE**

(56) Documents cités :
**US A 4 031 893**
**US A 4 177 810**

(73) Titulaire : **Laboratoires POS**
**F-68240 Kaysersberg (FR)**

(72) Inventeur : **Anderman, Guy**
**38, Avenue Clémenceau**
**F-68000 Colmar (FR)**
Inventeur : **Erhart, Michel**
**13, Place de ia Mairie**
**F-68250 Pfaffenheim (FR)**
Inventeur : **Mergel, Dominique**
**1, Place du 19 décembre**
**Sigolsheim F-68240 Kaysersberg (FR)**

(74) Mandataire : **Cuer, André**
**CABINET CUER 30, rue de Léningrad**
**F-75008 Paris (FR)**

Jouve, 18, rue St-Denis, 75001 Paris, France

## Dispositif semi-automatique pour le gavage d'animaux

La présente invention a trait aux dispositifs d'injection du fluide et se rapporte plus particulièrement à un appareil semi-automatique de gavage d'animaux, notamment de laboratoire.

Dans la plupart des laboratoires, le gavage s'effectue le plus souvent manuellement à l'aide d'une seringue munie d'une canule utilisée successivement pour tous les animaux. Si l'un deux présente une infection, les germes sont transmis à tous les autres. Pour certaines études, il est indispensable d'éviter toute transmission de germes d'un animal à un autre, même en l'absence de contamination par un agent pathogène. Jusqu'à présent, on a donc utilisé, pour chaque animal, une canule et une seringue différentes. Il en résulte une grande consommation de canules et de seringues, ce qui augmente le coût du gavage, et une perte de temps importante.

Par ailleurs, on connaît des distributeurs de réactifs constitués d'une seringue adaptée au volume que l'on veut délivrer, d'un moteur électrique actionnant le piston de la seringue et d'une vanne automatique à trois voies qui met la seringue en relation alternativement avec le tuyau de distribution et celui d'aspiration. Lorsque l'appareil est mis en route, le moteur pousse le piston, dans le corps de la seringue et le contenu de celle-ci est chassé dans le tuyau de distribution. On peut utiliser ces distributeurs pour le gavage d'animaux lorsqu'on adapte une canule ou tuyau de distribution. Mais avec ces appareils, il est nécessaire de changer la canule pour chaque animal.

On connaît également des systèmes d'injection de fluide, comme, par exemple, des seringues pour l'injection hypodermique de médicament dans lesquelles un piston à ressort appuie sur la cartouche de médicament et permet de délivrer un volume donné de fluide (US-A-4 031 893). Toutefois, de tels systèmes ne sont pas adaptés à l'introduction de masses liquides ou pâteuses dans l'estomac d'animaux du fait, notamment, de l'aseptisation nécessaire lors d'injections réitérées avec la même seringue.

L'invention a pour but de proposer un dispositif d'injection, utilisable notamment pour le gavage d'animaux de laboratoire, dans lequel l'aseptisation du moyen d'injection est faite à chaque opération et où les étapes de prélèvement de matière, d'injection et d'aseptie sont automatisées, avec utilisation en permanence de la même canule (pour le gavage) ou de la même aiguille (piqures en série).

Le nouvel appareillage selon l'invention est caractérisé en ce qu'il comporte la combinaison des moyens suivants : une seringue mue par piston pour prélever et délivrer le volume de chaque gavage à l'animal par l'intermédiaire d'une canule destinée à pénétrer par mise en place manuelle dans l'estomac de l'animal ; un support dans lequel est placée manuellement la canule et qui est relié à des canalisations mises en débit par un contacteur placé sur le support et actionné par la mise en place manuelle de la canule pour assurer un cycle de rinçage et d'aseptie de la canule à l'intérieur et à l'extérieur de celle-ci avec commandes par séries de vannes ; un motoréducteur actionnant un chariot mobile sur rails pour assurer les déplacements du piston dans la seringue et dont l'arbre est munie de cames pour assurer la commande ainsi que l'ouverture et la fermeture desdites vannes de prélèvement et injection de fluide de gavage ; un ensemble de programmation muni de cames de commande desdites vannes et destiné à assurer les opérations automatiques de prélèvement de liquide, remplissage de la seringue, rinçage et d'aseptie de la canule, les seules manœuvres manuelles étant la mise en place de la canule dans l'estomac de l'animal ainsi que sa pose dans le support de rinçage d'aseptie après chaque injection de gavage.

La description ci-dessous en regard des dessins annexés, permettra de bien comprendre comment l'invention peut être mise en pratique dans le cas, non limitatif, du gavage d'animaux.

La figure 1 est un schéma général d'un dispositif selon l'invention.

La figure 2 est un schéma électrique d'alimentation et de commande du dispositif selon l'invention.

La figure 3 est une vue de dessus du dispositif selon l'invention.

La figure 4 est une vue en coupe selon la ligne IV-IV de la figure 3.

La figure 5 est une vue du support recevant le moyen d'injection.

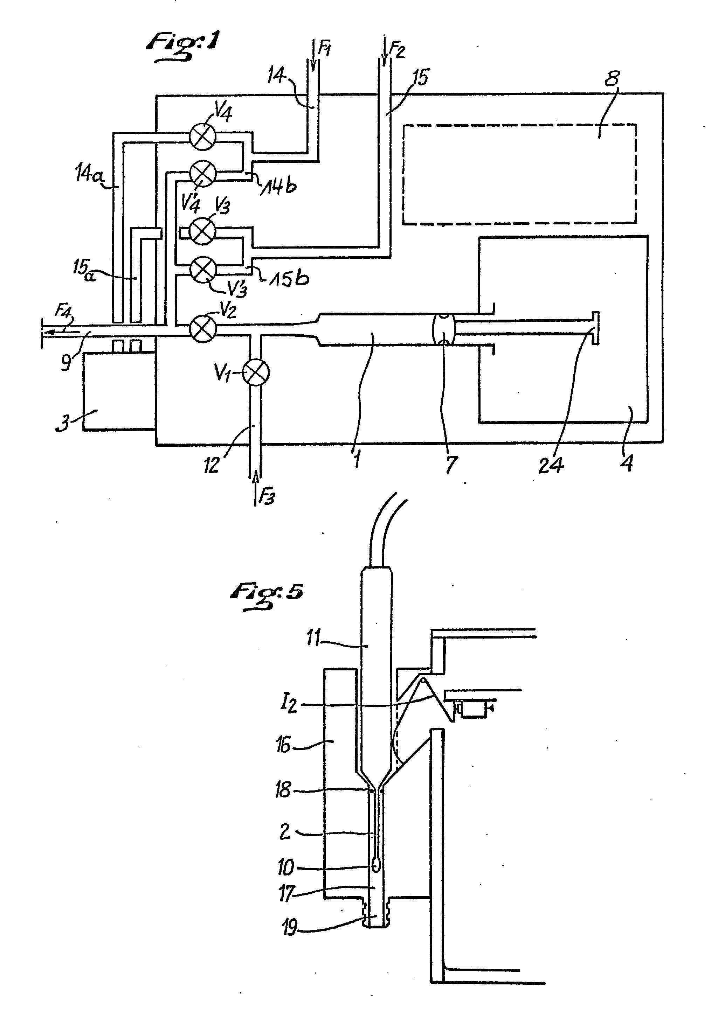

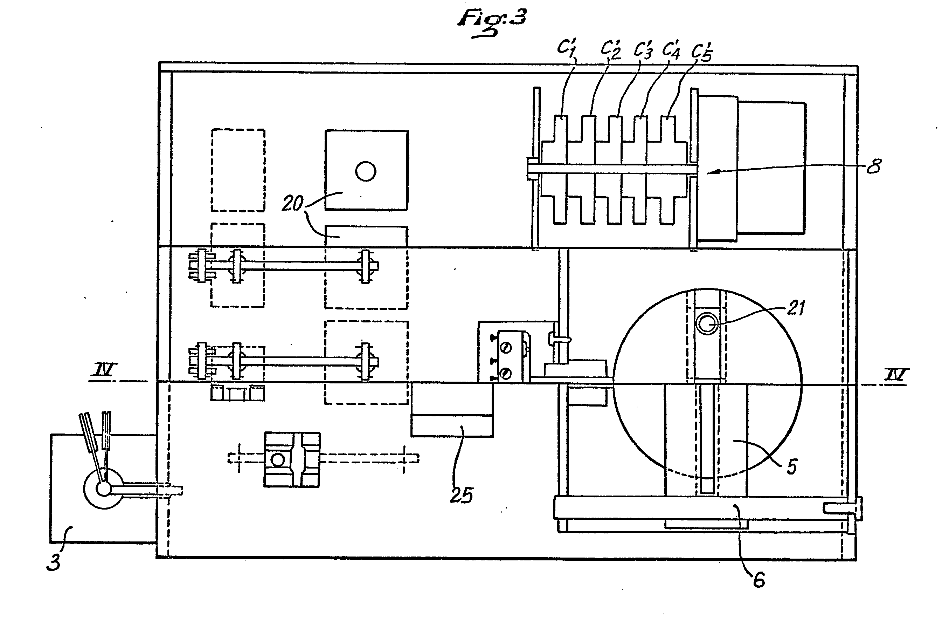

Comme on le voit sur les figures 1 et 5 le dispositif selon l'invention est constitué d'une seringue 1 et d'une canule 2, d'un support 3 recevant la canule pendant les opérations de rinçage et d'aseptie, d'un motoréducteur 4 actionnant un chariot 5 (voir figure 3) mobile sur deux rails 6, ce qui assure les déplacements du piston 7 dans le corps de la seringue 1 amovible. En outre un ensemble de vannes $V_1$, $V_2$, $V_3$, $V'_3$, $V_4$, $V'_4$ électromagnétiques permet de délivrer en temps voulu, les différents liquides dans la canule et hors de celle-ci. Enfin, un programmateur 8 commande les électro-vannes, c'est-à-dire les différentes étapes de rinçage et d'aseptie de la canule et le motoréducteur.

La seringue 1 et la canule 2 sont reliées par des tuyaux 9 et des raccords (non représentés). La seringue détermine le volume délivré à chaque injection, de préférence chaque gavage, par son diamètre et la course du piston. La canule 2 de gavage, est destinée à pénétrer dans l'estomac de l'animal et est constituée d'un tube mince, de préférence en acier inoxydable, muni à une extrémité d'une olive 10 pour ne pas blesser l'animal, et à l'autre extrémité d'une poignée 11 servant à tenir l'ensemble en main. Le fluide de gavage pénètre dans la seringue par le tuyau de remplissage 12, un tuyau de gavage 9 relie directement la seringue 1 à la canule 2. Le tuyau 14 permet l'admission d'alcool dans le support (3) de canule, par le tranchement 14a et l'admission d'alcool à l'intérieur de la canule, par le branchement 14b. De même le tuyau 15 permet l'admission d'eau dans le support (3) de canule, par le branchement 15a et l'admission d'eau à l'intérieur de la canule, par le branchement 15b. L'ensemble du circuit est amovible, de manière à pouvoir être nettoyé, stérilisé ou remplacé. L'alcool se déplace dans le sens de la flèche $F_1$, l'eau dans le sens de la flèche $F_2$ et ceci grâce à des pompes placées en amont. De préférence, le circuit de gavage est amovible, de manière à pouvoir être nettoyé et stérilisé.

Le support 3 de canule est constitué d'un bloc 16, de préférence en acier inoxydable, percé d'un canal central 17, cylindrique et de dimension convenable pour que la canule 2 puisse y pénétrer. Un contacteur $I_2$ incorporé dans le bloc assure, dès que la canule est posée dans le support, la mise en route du cycle de rinçage et d'aseptie de la canule, comme on le décrira plus en détail ci-dessous. Deux petits orifices 18 débouchent dans le canal central, à la base de la poignée 11, et permettent à l'eau et l'alcool envoyés dans la canule, de s'échapper de celle-ci.

Pendant le rinçage et l'aseptie, l'alcool ou l'eau provenant respectivement des branchements 14a, 14b ou 15a, 15b, s'écoule à la fois à l'intérieur et à l'extérieur de la canule et est en contact avec ses parois internes et externes, ce qui assure une désinfection et un nettoyage complet. Au bas du support, un téton 19 permet le raccordement d'un tuyau d'évacuation.

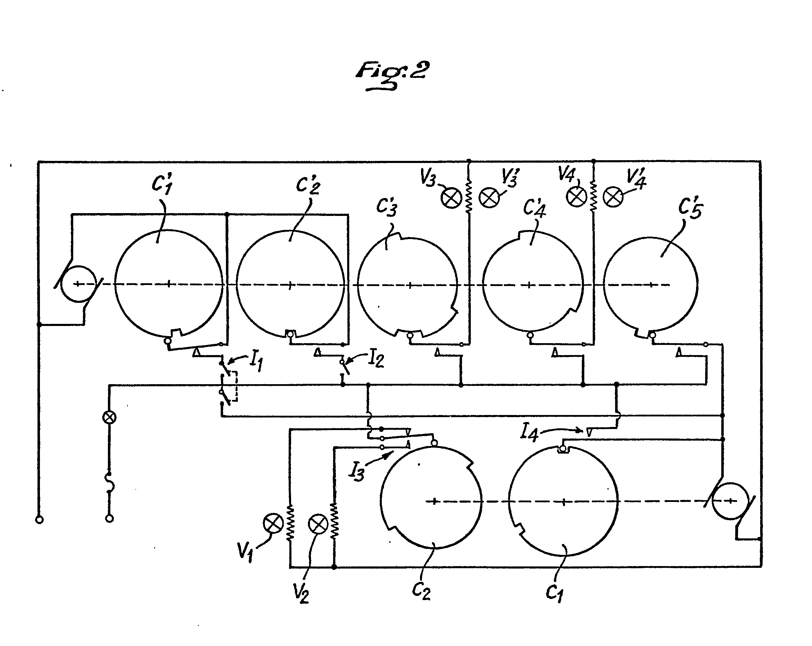

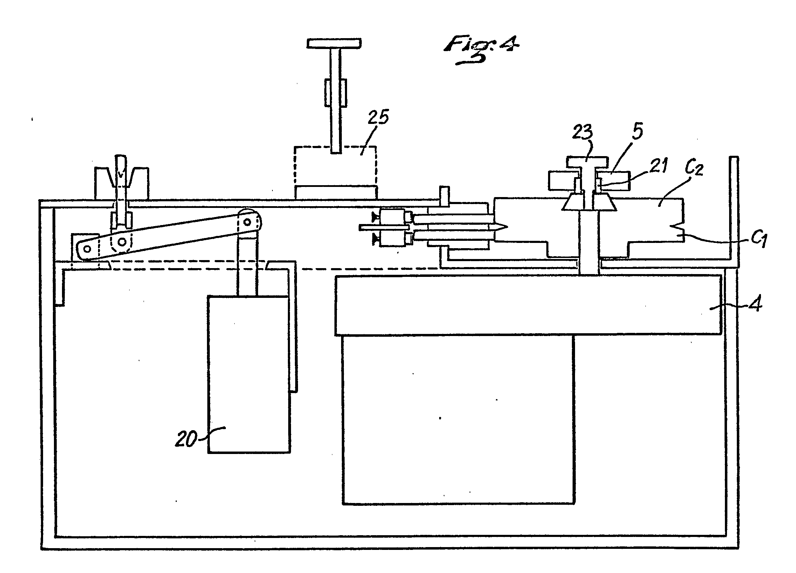

Le piston 7 de la seringue est animé par un motoréducteur 4 muni d'un dispositif de freinage par électro-aimant 20 (figure 4) assurant son arrêt immédiat lorsque l'alimentation électrique est coupée. Sur l'arbre du motoréducteur, sont montées deux cames $C_1$ et $C_2$. La came $C_1$ (figure 2) assure, par l'intermédiaire du contacteur $I_4$, l'alimentation électrique du moteur, lorsque la commande de démarrage cesse, ainsi que l'arrêt du moteur à la fin du prélèvement ou de l'injection, c'est-à-dire du gavage. La deuxième came $C_2$ (figure 2) agit sur un inverseur $I_3$ qui commande l'ouverture ou la fermeture des vannes $V_1$ et $V_2$, en fonction de la position du piston 7.

Le mouvement de rotation est transformé en mouvement longitudinal alternatif par un chariot 5 coulissant sur deux rails 6 cylindriques, sous l'action d'un excentrique 21, monté sur un disque entraîné par le motoréducteur. L'excentrique peut coulisser suivant un rayon de son disque support. Ainsi, l'amplitude du mouvement peut être modifiée en fonction de la seringue utilisée ou de la course désirée. On peut donc injecter un volume prédéterminé. L'excentrique est immobilisé sur le disque à l'aide d'une vis à molette 23. Le talon 24 du piston est fixé sur le chariot 5 qui l'entraîne. De plus, la seringue est fixée solidement sur le dispositif par le berceau 25.

Les vannes électromagnétiques permettent de délivrer en temps voulu le liquide désiré. La vanne $V_1$ commande l'ouverture ou la fermeture du circuit d'aspiration, dans le sens de la flèche $F_3$, du liquide de gavage. La vanne $V_2$ commande l'injection de la solution de gavage, selon la direction de la flèche $F_4$, dans la canule. Les vannes $V_3$ et $V'_3$ commandent l'injection de l'eau de rinçage dans le canal du support de canule et dans la canule. De même, les vannes $V_4$ et $V'_4$ commandent l'injection d'alcool dans le canal du support de canule et dans la canule.

Le programmateur 8 commande les différentes étapes de rinçage, d'aseptie de la canule ainsi que le remplissage de la seringue (figures 2 et 3). Il est constitué d'un ensemble de cames agissant chacune sur un contacteur. Les cames $C'_1$ et $C'_2$ assurent l'alimentation électrique du programmateur. La came $C'_3$ commande les vannes $V_3$ et $V'_3$ donc le rinçage à l'eau. La came $C'_4$ commande les vannes $V_4$ et $V'_4$, donc l'aseptie à l'alcool. La came $C'_5$ commande le démarrage du motoréducteur 4, donc le remplissage de la seringue.

Le fonctionnement du dispositif selon l'invention est le suivant. Lorsque la canule 2 est en place dans l'estomac de l'animal, l'action du pied sur le contacteur double $I_1$ commande le gavage. Le motoréducteur 4 se met en route et repousse le piston 7 dans le corps de la seringue. Lorsque l'action du pied sur le contacteur $I_1$ cesse, le motoréducteur 4 continue de tourner, le courant passant grâce au contacteur $I_4$ actionné par la came $C_1$. La vanne $V_2$ étant ouverte, et les vannes $V_1$, $V_3$ et $V_4$ étant fermées, le contenu de la seringue s'écoule dans le sens de la flèche $F_4$ (figure 1), puis par la canule et va dans l'estomac. Lorsque le piston arrive en fin de course, le gavage est terminé. La came $C_1$ et le contacteur $I_4$ coupent l'alimentation électrique du motoréducteur 4. La came $C_2$ et l'inverseur $I_3$ commandent la fermeture de la vanne $V_2$ et l'ouverture de la vanne $V_1$. L'action du pied sur le contacteur double $I_1$ assure également l'avance d'un pas du programmateur 8. L'ensemble contact-came $C'_2$ se ferme. Le programmateur cesse d'avancer quand l'ensemble contact-came $C'_1$ s'ouvre.

Lorsque le gavage est terminé, la canule 2 est retirée de l'estomac de l'animal et mise en place dans le support 3. Le contacteur $I_2$ se ferme alors. Le programmateur 8 se met en route et commande successivement et dans l'ordre les opérations suivantes : L'ensemble contact-came $C'_1$ se ferme et est prêt à alimenter le programmateur lors du gavage suivant. L'ensemble contact-came $C'_3$ se ferme puis s'ouvre ce qui commande l'ouverture des vannes $V_3$ et $V'_3$. L'intérieur et l'extérieur de la canule sont rincés à l'eau. L'ensemble contact-came $C'_4$ se ferme puis s'ouvre, ce qui commande l'ouverture des vannes $V_4$ et $V'_4$. L'intérieur et l'extérieur de la canule sont rincés à l'alcool et sont aseptisés. L'ensemble contact-came $C'_3$ se ferme et s'ouvre, ce qui commande l'ouverture des vannes $V_3$ et $V'_3$. L'intérieur et l'extérieur de la canule 2 sont rincés à l'eau. Puis, pendant un bref instant, l'ensemble contact-came $C'_5$ est fermé, ce qui commande le démarrage du motoréducteur 4. Enfin, l'ensemble contact-came $C'_2$ est ouvert, le programmateur 8 n'est plus alimenté et s'arrête.

Après son démarrage, le motoréducteur 4 continue à tourner car il est alimenté par l'ensemble came $C_1$-contacteur $I_4$. Le piston 7 se retire du corps de la seringue. Celle-ci se remplit de solution de gavage qui circule dans le sens de la flèche $F_3$ (figure 1). Lorsque le piston arrive en bout de course, l'ensemble inverseur $I_3$-came $C_2$ commande la fermeture de la vanne $V_1$ et l'ouverture de la vanne $V_2$. Le piston 7 est à nouveau repoussé dans le corps de la seringue 1. La solution de gavage est chassée dans la canule où elle remplace l'eau du rinçage précédent. Lorsque le piston 7 a parcouru une partie de course suffisante pour purger le tuyau 9 et la canule 2, l'ensemble contac-came $C_1$ s'ouvre et le motoréducteur 4 s'arrête. Le dispositif est prêt pour le gavage suivant.

Par conséquent, les opérations successives sont les suivantes : la canule est mise en place dans l'estomac de l'animal et la solution de gavage est chassée de la seringue, puis la canule est posée dans le support. L'intérieur et l'extérieur de la canule sont rincés une première fois, puis aseptisés à l'alcool et enfin rincés une deuxième fois. La seringue se remplit alors de la solution de gavage et celle-ci est chassée dans la canule où elle remplace l'eau de rinçage.

De préférence, le cycle de rinçage dure 15 secondes. La commande d'avance du programmateur dure 1 seconde, le premier rinçage à l'eau dure 3 secondes, l'aseptie à l'alcool dure 5 secondes, le deuxième rinçage à l'eau dure 5 secondes et le démarrage de l'aspiration de la solution de gavage dure 1 seconde. Pour obtenir ces laps de temps, la vitesse de rotation des cames est de 4 tours/minute.

Bien que la description se réfère essentiellement au cas d'application pratique du gavage d'animaux, le procédé et le dispositif selon l'invention sont susceptibles de nombreuses autres applications, notamment dans le cas de toutes injections de fluide dans une matière avec étape d'aseptisation.

## Revendication

Dispositif semi-automatique pour le gavage d'animaux, par prélèvement de fluide liquide ou pâteux puis injection orale ou parenthale dans l'animal avec la même canule (2) ou seringue (1), caractérisé en ce qu'il comporte la combinaison des moyens suivants : une seringue (1) mue par piston (7) pour prélever et délivrer le volume de chaque gavage à l'animal par l'intermédiaire d'une canule (2) destinée à pénétrer par mise en place manuelle dans l'estomac de l'animal ; un support (3) dans lequel est placé manuellement la canule (2) et qui est relié à des canalisations (14-14a, 15-15a) mises en débit par un contacteur ($I_2$) placé sur le support et actionné par la mise en place manuelle de la canule (2), pour assurer un cycle de rinçage et d'aseptie de la canule à l'intérieur et à l'extérieur de celle-ci avec commandes par séries de vannes ($V_1$ à $V_4$) ; un motoréducteur (4) actionnant un chariot (5) mobile sur rails (6) pour assurer les déplacements du piston (7) dans la seringue (1) et dont l'arbre est munie de cames ($C_1$, $C_2$) pour assurer la commande ainsi que l'ouverture et la fermeture des vannes ($V_1$, $V_2$) de prélèvement et injection de fluide de gavage ; un ensemble de programmation (8) muni de cames ($C'_1$ à $C'_5$) de commande desdites vannes et destiné à assurer les opérations automatiques de prélèvement de liquide, remplissage de la seringue, rinçage et d'aseptie de la canule, les seules manœuvres manuelles étant la mise en place de la canule (2) dans l'estomac de l'animal ainsi que sa pose dans le support (3) de rinçage d'aseptie après chaque injection de gavage.

## Claim

Semi-automatic apparatus for cramming animals, by taking a liquid or pasty fluid then oral or parenthal injecting it in the animal with the same nozzle (2) or syringe (1), characterized by the combination of the following means : a syringe (1) moved by piston (7) for taking and delivering the volume of each cramming bolus to the animal through a nozzle (2) able to be manually put in the animal's stomach ; a support (3) in which the nozzle (2) is manually introduced and which is connected with canalisations (14-14a, 15-15a) set in flowing by a contactor (12) put on the support and driven by manually placing the nozzle (2) in order to ensure a rinsing and aseptis cycle of the nozzle inside and outside of it by control by means of series of flood-gates ($V_1$ to $V_4$) ; a motor-reducer (4) actuating a carriage (5) movable on rails (6) to cause the shifting of the piston (7) in the syringe (1) and of which the shaft is provided with cams ($C_1$, $C_2$) to control the opening and the closing of the flood-gates ($V_1$, $V_2$) for taking and injecting the cramming's fluid ; a programming unit (8) fitted with cams ($C'_1$ to $C'_5$) for controlling the said flood-gates and intended to insure the automatic operations of : taking of fluid, filling up of the syringe, rinsing and aseptis of the nozzle ; the manual operations alone being the putting in position of the nozzle (2) in the stomach of the animal and the placing of it in the support (3) of rinsing for aseptis after each cramming's injection.

## Anspruch

Halbautomatische Vorrichtung zum Stopfen von Tieren durch Entnahme von flüssigem oder pastenförmigem Fluid, danach orale oder parenterale Einspritzung in das Tier mit der gleichen Kanüle (2) oder Spritze (1), gekennzeichnet dadurch, daß sie aus der Kombination der folgenden Mittel besteht : einer Spitze (1), bewegt durch Kolben (7), zur Entnahme und zur Verabreichung der Menge einer jeden Stopfung am Tier mittels einer Kanüle (2), die dazu bestimmt ist, durch manuelle Einstellung in den Magen des Tieres einzudringen ; einem Halter (3), in den die Kanüle (2) manuell eingesetzt wird und der angeschlossen ist an Leitungen (14-14a, 15-15a), die zum Ausbringen gebracht werden durch einen Einschalter (12), der auf dem Halter angebracht ist und betätigt wird durch die manuelle Einstellung der Kanüle (2), um einen Spülungs- und Asepsiskreislauf im Inneren und Äußeren derselben zu gewährleisten mit Steuerungen von Reihenventilen ($V_1$ bis $V_4$) ; einem Motorgetriebe (4), das einen auf Schienen (6) verschiebbaren Schlitten (5) betätigt, um die Bewegungen des Kolbens (7) in der Spritze (1) zu gewährleisten und dessen Welle mit Zapfen ($C_1$, $C_2$) versehen ist zur Sicherstellung der Steuerung und des Öffnens und Schließens der Ventile ($V_1$, $V_2$) zur Entnahme und zur Einspritzung des Stopffluids ; einer Programmierungskonstruktion (8), versehen mit Zapfen ($C'_1$ bis $C'_5$) zur Steuerung der besagten Ventile und dazu vorgesehen, die automatischen Arbeitsgänge der Entnahme der Flüssigkeit, Füllen der Spritze, Spülung und Asepsis der Kanüle zu gewährleisten, die einzigen manuellen Betätigungen bestehen im Einsetzen der Kanüle (2) in den Magen des Tieres sowie ihr Einsetzen in den Halter (3) zur Asepsisspülung nach jeder Stopfeinspritzung.

Fig:1

Fig:5

Fig:2

Fig.3

Fig. 4